Europäisches Patentamt

European Patent Office

Office européen des brevets

(11) Publication number: **0 069 399**
**B1**

# EUROPEAN PATENT SPECIFICATION

(45) Date of publication of patent specification: **29.10.86**

(51) Int. Cl.⁴: **A 61 K 9/50**

(21) Application number: **82106124.9**

(22) Date of filing: **08.07.82**

(54) **Pharmaceutical composition containing ubidecarenone containing liposomes.**

(30) Priority: **08.07.81 JP 105689/81**

(43) Date of publication of application:
**12.01.83 Bulletin 83/02**

(45) Publication of the grant of the patent:
**29.10.86 Bulletin 86/44**

(84) Designated Contracting States:
**DE FR GB IT**

(56) References cited:
**FR-A-2 472 384**

**BULLETIN OF THE CHEMICAL SOCIETY OF JAPAN, vol. 53, October 1980, pages 2773-2777, Tokyo (JP); J. SUNAMOTO et al.: "Liposomal membranes. IV. Fusion of liposomal membranes induced by several lipophilic agents"**

**CHEMICAL ABSTRACTS, vol. 84, no. 21, 24th May 1976, page 201, no. 146509d, Columbus Ohio (USA); A.ALBERT et al.: "Mixed monolayers of coenzyme Q and natural and synthetic phospholipids"**

(73) Proprietor: **Eisai Co., Ltd.**
**6-10, Koishikawa 4-chome Bunkyo-ku Tokyo 112 (JP)**

(72) Inventor: **Takada, Masahiro**
**Eisai Shizan-Ryo 4-19-13, Kasuga Yatabe-machi Tsukuba-gun Ibaraki Prefecture (JP)**
Inventor: **Yuzuriha, Teruaki**
**4920, Kidamari-machi Tsuchiura-shi Ibaraki Prefecture (JP)**
Inventor: **Katayama, Kouichi**
**33-8, Umezono 2-chome Sakura-mura Niibari-gun Ibaraki Prefecture (JP)**
Inventor: **Sunamoto, Junzo**
**4-16-10, Yokoo Nagasaki-shi Nagasaki Prefecture (JP)**

(74) Representative: **Hansen, Bernd, Dr.rer.nat. et al Hoffmann, Eitle & Partner Patentanwälte Arabellastrasse 4 D-8000 München 81 (DE)**

(56) References cited:

**CHEMICAL ABSTRACTS, vol. 95, no. 11, 14th September 1981, page 220, no. 92725c, Columbus Ohio (USA); M. ESPOSTI et al.: "Incorporation of ubiquinone homologs into lipid vesicles and mitochondrial membranes"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to a pharmaceutical composition containing ubidecarenone-containing liposomes, which permits rapid transfer of ubidecarenone from the blood to a target organ.

Ubidecarenone, also known as coenzyme $Q_{10}$, has recently come into widespread clinical use as a medicine effective for improving the function of the heart.

There is still room for improvement in the speed of transfer of this substance from the blood to a target organ in intravenous or oral administration. Since the aforesaid substance is a medicine intended for improving the function of the heart, it is desired that the speed of its transfer to the target organ in the initial stage and the amount of its transfer be great. However, if this substance is formed into a pharmaceutical preparation in accordance with a conventional technique, the speed of its absorption from the blood is very slow, and therefore the speed of its transfer to the target organ and the amount transferred are reduced.

Since ubidecarenone is a normally solid lipid-soluble substance having a melting point of 48 to 52°C, it must be solubilized by a conventional technique involving the use of a surfactant such as HCO (polyoxyethylene-hardened castor oil) in order to render it convenient for intravenous administration. However, if it is administered in the thus solubilized state, the speed of absorption of ubidecarenone from the blood is low, and the speed of its transfer in the initial stage to the heart, spleen and liver, i.e., the target organs, especially to the heart is low.

In the Bulletin of the Chemical Society of Japan, Vol. 53, pages 2773—2777, 1980, and Chemical Abstracts, Vol. 84, Abstract No. 156509d, 1976, there are disclosed ubidecarenone-containing liposomes.

In view of the above background, the present inventors have made various investigations about ubidecarenone-containing pharmaceutical compositions which are effective for increasing the transfer of ubidecarenone from the blood to predetermined target organs.

Thus, according to this invention there is provided a pharmaceutical composition containing ubidecarone-containing liposomes, the ubidecarone being contained in the membranes of the liposomes and the liposomes basically comprising phospholipids and sterols, as the active ingredient together with pharmaceutically acceptable carriers and/or diluents.

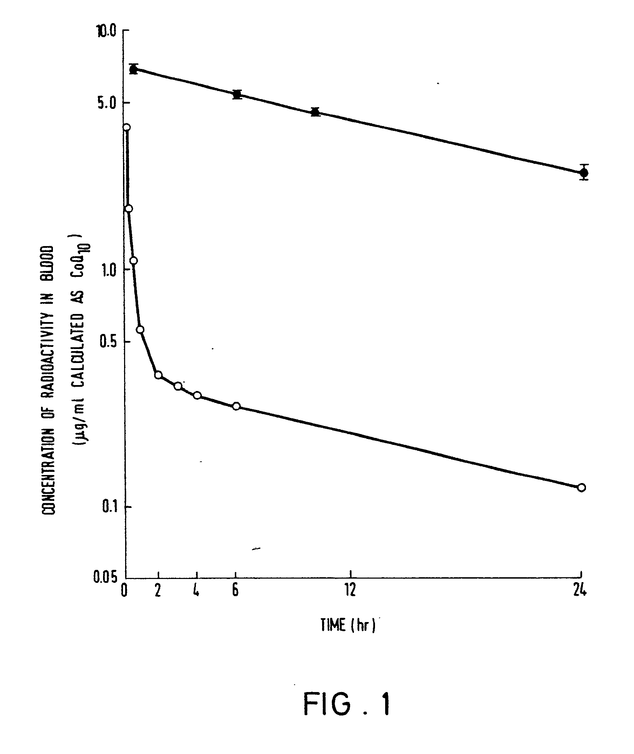

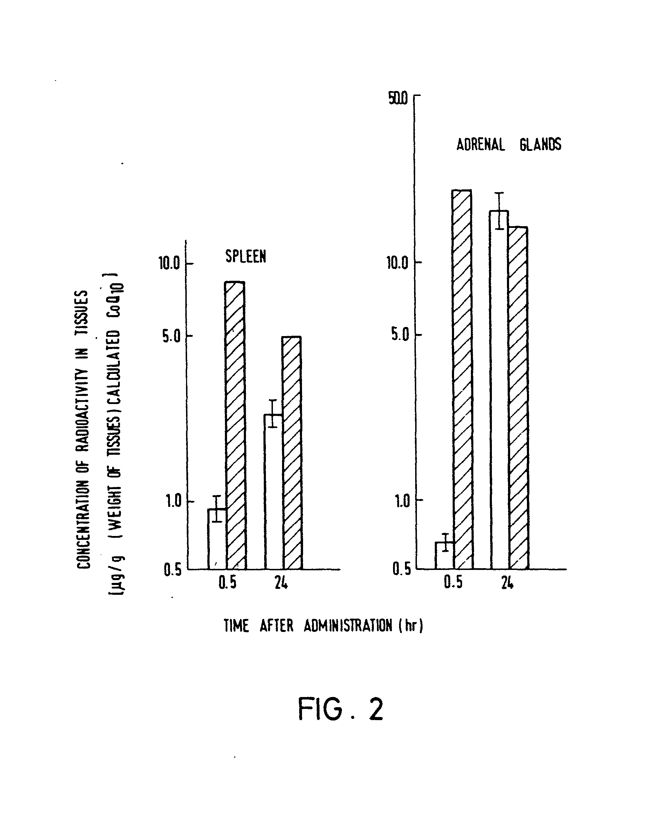

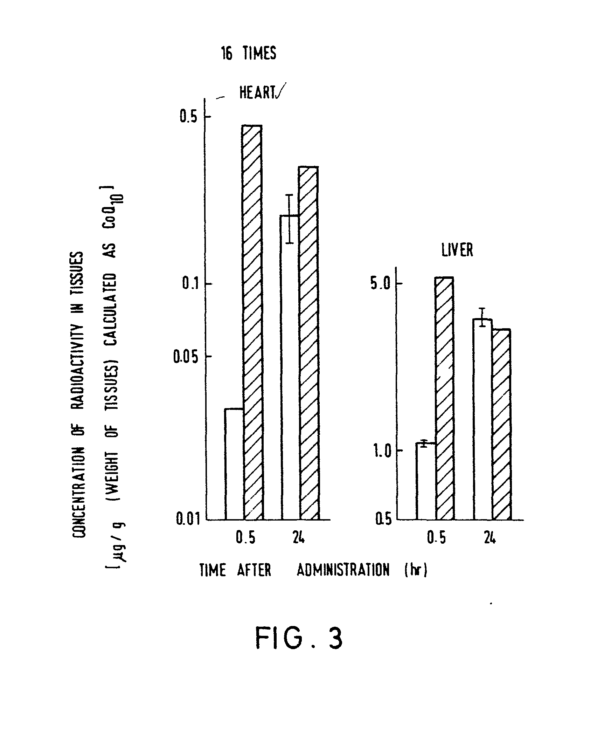

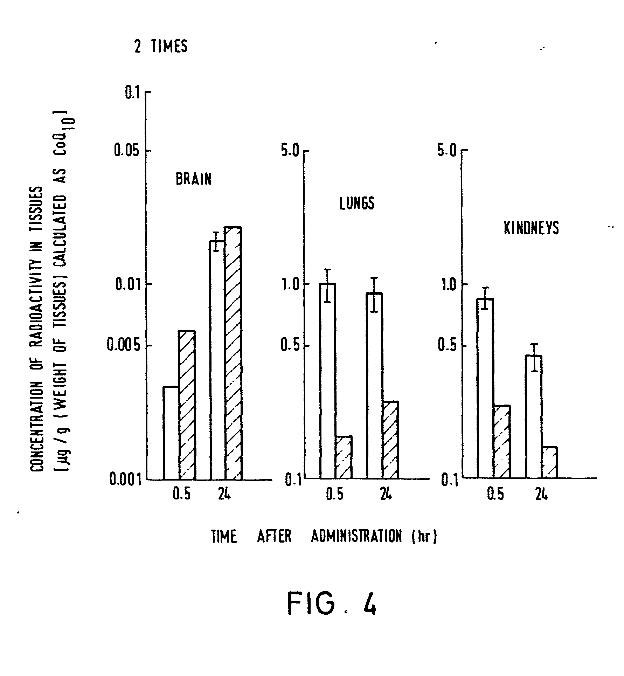

Figure 1 is a graphic representation showing the results obtained in Experimental Example 1 hereinafter, and Figures 2 to 4 are graphic representations showing the results obtained in Experimental Example 2 hereinafter.

In the Experimental Examples it is shown that after administration of the pharmaceutical composition of this invention, the speed of disappearance of ubidecarenone from the blood was much higher and the amount of ubidecarenone transferred within a predetermined period to organs, except the lungs and kidneys, was larger, as compared with the administration of the solubilized ubidecarenone prepared in accordance with conventional techniques.

Generally, liposomes are known, and a conventional method is available for their production. For the first time in accordance with this invention, a pharmaceutical composition comprising ubidecarenone-containing liposomes has been produced, thereby solving the aforesaid basic problem of ubidecarenone and greatly increasing the usefulness of ubidecarenone.

In the pharmaceutical composition of this invention, ubidecarenone is contained in the membranes constituting the liposomes. The liposomes are composed basically of phospholipids and sterols. Ubidecarenone is present together with these substances and dispersed uniformly within the membranes.

Examples of the phospholipids used in this invention are phosphatidyl choline, phosphatidyl ethanolamine, phosphatidyl serine, sphingomyelin, phosphatidyl, glycerol, phosphatidic acid, phosphatidyl inositol and mixtures thereof. It is, however, not specifically limited to these exemplified species. On the other hand, cholesterol is most preferred as the sterol.

The proportions of ubidecarenone, the phospholipid and the sterol in the liposomes are preferably such that at least 10 moles of the phospholipid and at least 1 mole of the sterol are present per mole of ubidecarenone. For example, per mole of ubidecarenone, the proportion of the phospholipid is preferably 10 to 30 moles, if it is egg yolk phosphatidyl choline, and the proportion of the sterol is preferably 1 to 10 moles, if it is cholesterol.

Preferred proportions in moles of various phospholipids and cholesterol per mole of ubidecarenone are shown in the following table.

| Examples | 1 | 2 | 3 | 4 | 5 | 6 | 7 |
|---|---|---|---|---|---|---|---|
| Phospholipid PC | 10 | 15 | 18 | 12 | 5 | 9 | 20 |
| PS | | | | | 5 | | |
| Cholesterol | 5 | 5 | 4 | 4 | 5 | 5 | 5 |

PC: phosphatidyl choline
PS: phosphatidyl serine

The liposomes when observed under an electron microscope, are spherical particles having a particle diameter of about 0.1 to 5.0 μm. Their lyophilized products are aggregated and in appearance are in the form of a block. When put into water or salt solutions, they disperse well to give a homogeneous solution.

The liposomes can be produced substantially in accordance with the conventional technique for the production of liposomes. For example, they can be produced by charging a membrane-constituting component of the liposomes and ubidecarenone into an elongated round-bottom flask, adding chloroform to dissolve these substances, then evaporating the solvent, peeling the resulting membrane by adding a glass bead and a suitable buffer, ultrasonicating the resulting solution, passing the treated solution through a column of Sephadex® or Sepharose®, collecting liposomes fractions, and removing the solvent. The removal of the solvent can be effected by, for example, lyophilization.

If the lyophilization is carried out for at least 3 hours under a pressure of not more than 2.6 mbar, the liposomes can be isolated as a powder.

As shown in a working example to be given hereinafter the lyophilization is desirably carried out for 5 hours under a pressure of 0.4 mbar, for example. The following examples illustrate the preparation of ubidecarenone-containing liposomes.

Experimental Example 1
Sample:—

Liposomes were prepared by the same method as described in Example 1 except that $^{14}C$—$CoQ_{10}$ was used instead of $CoQ_{10}$ described in Example 1. The resulting liposomes were used as an assay sample. A control sample was obtained by adding HCO-60 to $^{14}C$—$CoQ_{10}$, the amount of the former being four times that of the latter, and subjecting the solution to ultrasonication, and adding physiological saline to a concentration of 0.6 mg/ml.

Procedure:—
1. Animal experiment

The assay sample was injected in an amount of 0.6 mg as $^{14}C$—$CoQ_{10}$/kg into the left femoral vein of male guinea pigs (body weight 300—350 g). The control sample was administered in the same dose by the same method. The animals were then left in a cage, and at predetermined periods of time, blood was drawn from the ear vein. The concentration of $^{14}C$—$CoQ_{10}$ in the blood was measured by the following method.

2. Measurement of radioactivity in the blood

20 μl or 50 μl of blood was taken from the ear vein, and solubilized with 0.75 ml of Soluene 350/isopropyl alcohol (1/1). Several drops of aqueous hydrogen peroxide were added to decolorize the solution. Then, 5 ml of Instagel/ 0.5N HCl (9/1) was added, and radioactivity was measured by means of a liquid scintillation counter.

3. Results

Variations with time in the radioactivity concentration of $^{14}C$—$CoQ_{10}$ in the blood after the administration of the assay sample or the control sample are shown in Figure 1.

In Figure 1, the curve marked by ○ refers the administration of the assay sample (average of two runs), and the curve marked by ● to the administration of the control sample (an average of 3 runs, and a standard deviation). As shown in Figure 1, the disappearance of $^{14}C$—$CoQ_{10}$ in the assay sample from the blood is indicated by two straight lines which are bent at 1 hour after the administration. The half-line period of its disappearance is 11.5 minutes in the first phase and 15.6 hours in the second phase. The disappearance of $^{14}C$—$CoQ_{10}$ in the control sample from the blood is indicated by one straight line, and the half-life period of its disappearance is 20.1 hours. It is seen from these results that the disappearance of $^{14}C$—$CoQ_{10}$ in the assay sample from the blood is much more rapid than that of $^{14}C$—$CoQ_{10}$ in the control sample.

Experimental Example 2
Samples:—

The same assay sample and control sample as described in Experimental Example 1 were used.

Procedure:—
1. Animal experiment

Each of the above samples was injected in a dose of 0.6 mg/kg into the left femoral vein of male guinea pigs (body weight 300 g—350 g). The

3

animals were then left in a cage. Thirty minutes and 24 hours respectively after the administration, the animals were killed by decapitation, and the organs were removed, in order to prevent contamination of the brain and heart by blood, physiological saline was circulated from the left ventricle to the jugular vein to draw off the blood, and then the brain and heart were removed.

## 2. Measurement of radioactivity in the tissues

About 100 mg of each organ was added to 0.5 ml of Soluene 350, and incubated at 50°C for 2 hours to dissolve the tissues. Then, 6 ml of Instagel/0.5N HCl (9/1) was added, and radioactivity was measured by using a liquid scintillation counter.

## 3. Results

Thirty minutes and 24 hours respectively after the intravenous injection of $^{14}C$—$CoQ_{10}$ in the assay sample and $^{14}C$—$CoQ_{10}$ in the control sample, the concentrations of radioactivity in the main organs were expressed in terms of $CoQ_{10}$, and the transfer of the former to the organs was compared with that of the latter. The results are shown in Figures 2 to 4.

In Figures 2 to 4, the hatched columns ▨ refer to the administeration of the assay sample (an average of 2 runs), and the blank columns ▢, to the administration of the control sample (an average of 3 runs, and a standard deviation, except that in the case of the results obtained 30 minutes after the administration with regard to the brain and heart, the indicated values are the average of 2 runs). In regard to the distribution of $^{14}C$—$CoQ_{10}$ in the tissues, its concentration increased more rapidly, and lasted longer, in the assay sample than in the control sample. Thus, the distribution of the assay sample differs from that of the control sample. In regard to the transfer of $^{14}C$—$CoQ_{10}$ to the brain, heart, liver, spleen and adrenal gland, it was distributed more rapidly in higher concentrations in the case of the assay sample than in the case of the control sample. It was specifically found that 30 minutes after the administration, $^{14}C$—$CoQ_{10}$ in the assay sample was distributed in the heart in a concentration 16 times as high as that in the control sample and in the brain twice as high as that in the control sample.

The following Examples illustrate the present invention further.

## Example 1

36 mg ($4.5 \times 10^{-5}$ mole) of egg yolk phosphatidyl choline, 5.85 mg ($1.5 \times 10^{-5}$ mole) of cholesterol and 2.16 mg ($2.5 \times 10^{-6}$ mole) of ubidecarenone, i.e. $CoQ_{10}$, were dissolved in 2 ml of chloroform. Chloroform was removed under reduced pressure by using a rotary evaporator. The resulting thin film was dried overnight in a desiccator under reduced pressure to removed chloroform completely. One glass bead and 3.0 ml of a buffer A (to be described below) were added twice to the thin film left on the bottom of the desiccator, and the mixture was shaken by a vortex mixer until the film was completely peeled off.

The solution was then transferred to a branched test tube, and subjected to ultrasonication treatment at 42W for 53 minutes by using a probe-type sonicator intermittently every 30 seconds in an ice bath under a stream of argon to give a translucent pale yellow solution.

The solution was applied to a column ($1.6 \times 45$ cm) of Sepharose® 4B, and eluted with the buffer A. Then, 1.8 ml portions of the eluate were taken respectively into 60 test tubes. A fraction which was eluted near the void volume of this column was concentrated to a final volume of 1.7 ml (0.55 mg as $CoQ_{10}$) by means of a polycarbonate membrane having a pore size of 0.08 μm. The resulting liposomes had a diameter of 80 to 100 μm by observation under an electron microscope.

The buffer A denotes a 0.01M phosphate buffer (pH 7.4) containing 0.1M NaCl.

## Example 2

A 200 ml elongated round-bottom flask was charged with 60.0 mg (3 moles) of egg yolk phosphatidyl choline, 9.8 mg (1 mole) of cholesterol and 6.5 mg (0.3 moles) of ubidecarenone, and 4 ml of chloroform was added to dissolve them. The solvent was evaporated under reduced pressure. Four milliliters of physiological saline and a glass bead were added, and dispersed by using a vortex mixer. The resulting multilayer film was transferred to a branched test tube, and under an atmosphere of nitrogen gas, subjected to ultrasonication treatment at 25 KW for 15 minutes in an ice bath. The solution was then charged onto a column of Sephadex® G-50 and eluted with physiological saline as an eluent. Liposome fractions were collected and diluted to 30 ml. The diluted liposome fractions were lyophilized for 5 hours under a pressure of 0.4 mbar.

## Claims

1. Pharmaceutical composition, characterized in that it contains ubidecarenone-containing liposomes, the ubidecarenone being contained in the membranes of the liposomes and the liposomes basically comprising phospholipids and sterols, as active ingredient together with pharmaceutically acceptable carriers and/or diluents.

2. Composition of claim 1 wherein the phospholipid is selected from phosphatidyl choline phosphatidyl ethanolamine, phosphatidyl serine, sphingomyelin, phosphatidylglycerol, phosphatidic acid, phosphatidyl inositol and mixtures thereof.

3. Composition of claim 1 or 2 wherein the sterol is cholesterol.

4. Composition of any of claims 1 to 3 which comprises 1 mole of ubidecarenone, at least 10 moles of phospholipid, and at least 1 mole of sterol.

**Patentansprüche**

1. Pharmazeutische Zusammensetzung, dadurch gekennzeichnet, dass sie Ubidecarenonenthaltende Liposome enthält, wobei das Ubidecarenon in den Membranen der Liposomen vorkommt und die Liposomen als Basis Phospholipide und Sterine als Wirkstoff zusammen mit pharmazeutisch annehmbaren Trägern und/oder Verdünnungsmitteln umfassen.

2. Zusammensetzung nach Anspruch 1, worin das Phospholipid ausgewählt ist aus Phosphatidylcholin, Phosphatidylethanolamin, Phosphatidylserin, Sphingomyelin, Phosphatidylglycerin, Phosphatidinsäure, Phosphatidylinosit und Gemische derselben.

3. Zusammensetzung nach Anspruch 1 oder 2, worin das Sterin Cholesterin darstellt.

4. Zusammensetzung nach einem oder mehreren der Ansprüche 1 bis 3, welche 1 Mol Ubidecarenon, mindestens 10 Mol Phospholipid und mindestens 1 Mol Sterin umfasst.

**Revendications**

1. Composition pharmaceutique, caractérisée en ce qu'elle contient des liposomes qui contiennent l'ubidécarénone, l'ubidécarénone étant contenue dans les membranes des liposomes et les liposomes comprenant fondamentalement des phospholipides et des stérols comme ingrédients actifs, avec des véhicules et/ou diluants pharmaceutiquement acceptables.

2. Composition pharmaceutique selon la revendication 1, dans laquelle le phospholipide est choisi parmi la phosphatidyl choline, la phosphatidyl éthanolamine, la phosphatidyl sérine, la sphingomyéline, le phosphatidyl glycérol, l'acide phosphatidique, le phosphatidyl inositol et leurs mélanges.

3. Composition selon la revendication 1 ou 2, dans laquelle le stérol est le cholestérol.

4. Composition selon l'une quelconque des revendications 1 à 3, qui comprend 1 mole d'ubidécarénone, au moins 10 moles de phospholipide et au moins 1 mole de stérol.

FIG . 1

1

FIG . 2

FIG. 3

FIG. 4